# FASCICULE DE BREVET EUROPEEN

(11) **EP 3 153 136 B1**
(45) Date de publication et mention de la délivrance du brevet: **02.09.2020**
(21) Numéro de dépôt: 15306562.8
(22) Date de dépôt: 05.10.2015
(51) Int. Cl.: A61F 5/56

(54) **DISPOSITIF DENTAIRE DYNAMIQUE**
DYNAMISCHE DENTALVORRICHTUNG
DYNAMIC DENTAL DEVICE

(43) Date de publication de la demande: 12.04.2017
(73) Titulaire: Otte, Christophe, 1205 Genève (CH)
(72) Inventeur: Otte, Christophe, 1205 Genève (CH)
(74) Mandataire: Brungard, Yves Francois

(56) Documents cités:
- WO-A1-91/03215
- WO-A1-2014/012827
- WO-A1-2014/139492

## Description

L'invention concerne un dispositif dentaire dynamique pour la rééducation de la mastication chez un patient.

En moyenne, au cours d'une journée, les mâchoires d'un être humain sont sollicitées dix mille fois. Or ces sollicitations quotidiennes surviennent dans le cadre d'un déséquilibrage pour quatre vingt pour cent de la population. Ce déséquilibre entre les deux maxillaires inférieur et supérieur formant la mâchoire est principalement causée par un trouble de la musculature et une mauvaise occlusion. Ainsi, le fait de serrer les dents et/ou de mastiquer des aliments dans un tel déséquilibre favorise des tensions asymétriques au niveau de la tête et du coup d'un patient et occasionne à la longue des troubles plus ou moins invalidants de la posture.

Il y a donc une nécessité de pouvoir rééduquer un patient atteint par un tel déséquilibre de manière simple et efficace.

Un dispositif selon le préambule de la revendication 1 est connu du document WO 2014/139492 qui divulgue une gouttière occlusale en forme de U en matériau thermoplastique destinée à empêcher les ronflements et l'apnée du sommeil obstructive.

A cette fin, il est prévu, selon l'invention, un dispositif dentaire, destiné à être inséré dans la bouche d'un patient entre des maxillaires supérieur et inférieur, comprenant un bloc de matériau déformable élastiquement, en forme en « U » et en forme de coin s'affinant vers les extrémités des branches de la forme en « U », comportant :
- sur une face supérieure, une première gouttière s'étendant le long de la forme en « U », conformée à recevoir en appui les dents du maxillaire supérieur
- sur une face inférieure, une deuxième gouttière s'étendant le long de la forme en « U », conformée à recevoir en appui les dents du maxillaire inférieur ;
l'une parmi les première et deuxième gouttières comportant un premier bossage s'étendant en saillie depuis un fond de ladite gouttière;

le dispositif comportant une cavité située sous le bossage, entre le bossage et un fond de l'autre parmi les première et deuxième gouttières.

Avantageusement, mais facultativement, le dispositif dentaire selon l'invention présente au moins l'une des caractéristiques techniques suivantes additionnelles :
- Le bossage est situé dans l'une des branches de la forme en « U » ;
- la cavité est remplie avec un deuxième matériau déformable élastiquement différent de celui du bloc déformable élastiquement ;
- l'autre parmi les première et deuxième gouttières comporte un deuxième bossage s'étendant en saillie depuis un fond de ladite gouttière, au droit du premier bossage ;
- un autre bossage est situé dans l'autre des branches de la forme en « U » ;
- une cavité est agencée sous l'autre bossage ;
- le dispositif dentaire comporte un orifice antérieur aménagé traversant dans le bloc au niveau d'un fond de la forme en « U » du bloc ;
- l'orifice antérieur est rempli avec un troisième matériau déformable élastiquement différent de celui du bloc déformable élastiquement ;
- le bossage supplémentaire s'étend en saillie depuis un fond de ladite gouttière et est situé au niveau d'un fond de la forme en « U ».
- le dispositif dentaire comporte en outre un voile de palais s'étendant depuis un bord de la gouttière supérieure ; et,
- le bloc est réalisé en un élastomère, notamment thermoplastique.

D'autres avantages et caractéristiques de l'invention apparaîtront lors de la description ci après d'un mode de réalisation. Aux dessins annexés :

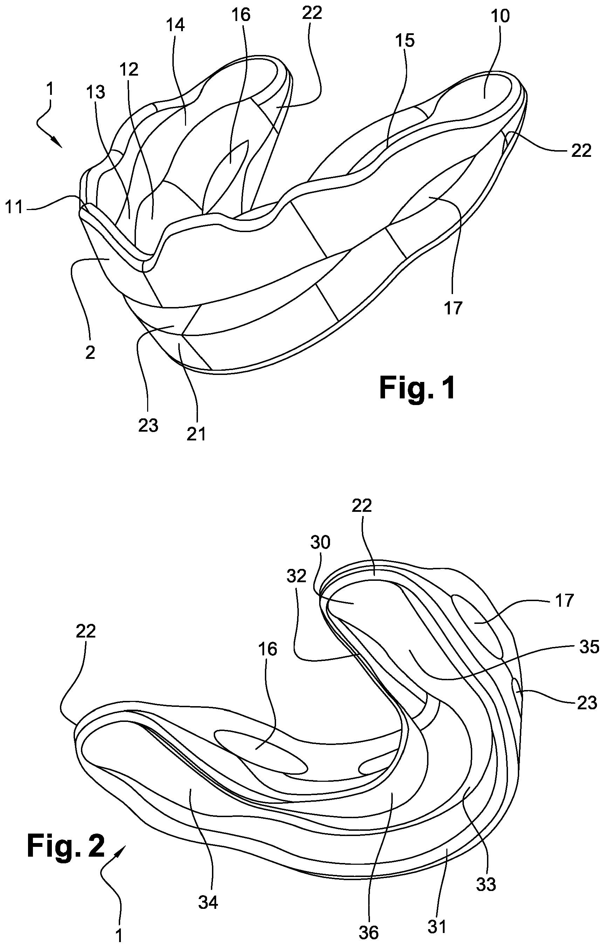

- les figures 1 et 2 sont des vues en trois dimensions d'un mode de réalisation d'un dispositif dentaire selon l'invention ;
- la figure 3 est une vue de dessus du dispositif dentaire des figures 1 et 2 ; et,
- la figure 4 est une vue de coté du dispositif dentaire des figures 1 et 2.

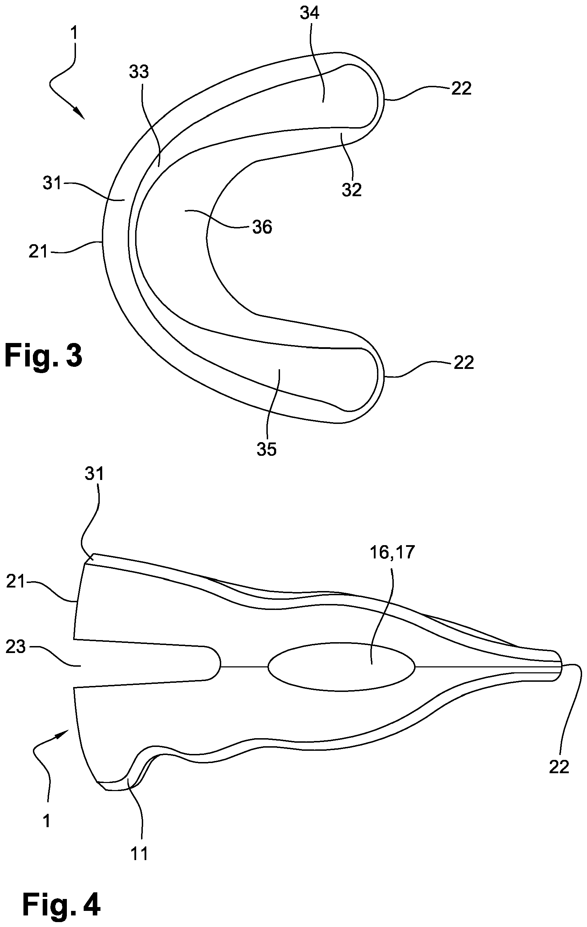

En référence aux figures, nous allons décrire un mode de réalisation d'un dispositif dentaire 1 selon l'invention. Le dispositif dentaire 1 selon l'invention comporte un bloc de matériau 2. Le bloc de matériau 2 présente en outre une forme sensiblement en « U » en vue de dessus, telle qu'illustrée en figure 3. Cette forme en « U » présente un fond 21 et deux branches 22. Le bloc de matériau 2 présente en outre globalement une forme de coin en vue de coté, comme cela est illustré en figure 4, qui vas en s'affinant depuis le fond 21 vers l'extrémité des branches 22. Une telle forme du dispositif dentaire 1 selon l'invention permet une insertion et un positionnement aisé dans la bouche d'un patient à traiter, entre les maxillaires supérieur et inférieur de ce dernier. La forme en « U » correspond à la forme des arches dentaires des maxillaires supérieur et inférieur. Le bloc de matériau 2 est réalisé avec un matériau tel que le bloc de matériau 2 puisse se déformer de manière élastique sous des efforts induits par des mouvements de la mâchoire du patient. Par exemple, le matériau est un élastomère, notamment thermoplastique. Le bloc de matériau 2 formant le dispositif dentaire 1 selon l'invention peut être obtenu par moulage. Il peut être aussi monobloc ou réalisé en plusieurs pièces assemblées par la suite.

Sur une surface supérieure, le dispositif dentaire 1 selon l'invention comporte une première gouttière 30 s'étendant le long de la forme en « U » du bloc de matériau 2, conformée à recevoir en appui les dents du maxillaire supérieur. Cette première gouttière 30 comporte un fond 33 et est délimitée par un bord extérieur 31 et un bord intérieur 32. Cela permet un bon positionnement du dispositif dentaire 1 selon l'invention sur l'arche dentaire du maxillaire supérieur du patient, les branches de la forme en « U » du bloc de matériau 2, et donc de la première gouttière 30 s'étendant en regard des molaires de l'arche dentaire du maxillaire supérieur. Au niveau de l'une des branches de la forme en « U », la première gouttière 30 comporte un premier bossage 34 s'étendant en saillie depuis le fond 31 de ladite première gouttière 30. De manière similaire, la première gouttière 30 comporte un deuxième bossage 35 s'étendant en saillie depuis le fond 31 de ladite première gouttière 30 et situé au niveau de l'autre des branches de la forme en « U ». De cette manière, le premier bossage 34, tout comme le deuxième bossage 35, s'étend, lors d'une utilisation, en regard de tout ou partie des molaires de l'arche dentaire du maxillaire supérieur. D'autre part, le dispositif dentaire 1 selon l'invention comporte un voile de palais 36 qui s'étend en saillie depuis le bord intérieur 32, sensiblement parallèlement à un plan de la surface supérieure du dispositif dentaire 1 selon l'invention. Le voile de palais 36 s'étend vers l'intérieur de la forme en « U » au niveau du fond 21. Le voile de palais 36 est conformé pour venir en appui le palais du patient, lors d'une utilisation du dispositif dentaire 1 selon l'invention.

Sur une surface inférieure, le dispositif dentaire 1 selon l'invention comporte une deuxième gouttière 10 s'étendant le long de la forme en « U » du bloc de matériau 2, conformée à recevoir en appui les dents du maxillaire inférieur. Cette deuxième gouttière 10 comporte un fond 13 et est délimitée par un bord extérieur 11 et un bord intérieur 12. Cela permet un bon positionnement du dispositif dentaire 1 selon l'invention sur l'arche dentaire du maxillaire inférieur du patient, les branches de la forme en « U » du bloc de matériau 2, et donc de la deuxième gouttière 10 s'étendant en regard des molaires de l'arche dentaire du maxillaire inférieur. Au niveau de l'une des branches de la forme en « U », la deuxième gouttière 10 comporte un premier bossage 14 s'étendant en saillie depuis le fond 11 de ladite deuxième gouttière 10. De manière similaire, la deuxième gouttière 10 comporte un deuxième bossage 15 s'étendant en saillie depuis le fond 11 de ladite deuxième gouttière 10 et situé au niveau de l'autre des branches de la forme en « U ». De cette manière, le premier bossage 14, tout comme le deuxième bossage 15, s'étend, lors d'une utilisation, en regard de tout ou partie des molaires de l'arche dentaire du maxillaire inférieur.

Le dispositif dentaire 1 selon l'invention comporte en outre une première cavité 16 située, ici, sous le premier bossage 34 de la première gouttière 30, entre le premier bossage 34 et le fond 11 de la deuxième gouttière 10, plus particulièrement entre le premier bossage 34 de la première gouttière 30 et le premier bossage 14 de la deuxième gouttière 10. De manière similaire, le dispositif dentaire 1 selon l'invention comporte en outre une deuxième cavité 17 située, ici, sous le deuxième bossage 35 de la première gouttière 30, entre le deuxième bossage 35 et le fond 11 de la deuxième gouttière 10, plus particulièrement entre le deuxième bossage 35 de la première gouttière 30 et le deuxième bossage 15 de la deuxième gouttière 10. La présence de la première 16 et/ou de la deuxième 17 cavité (s) va permettre de moduler, d'adapter, lors d'une utilisation du dispositif dentaire 1 selon l'invention, la déformation élastique du bloc de matériau 2 au niveaux du ou deus bossages 14,15,34,35. Ainsi, la première 16 et/ou la deuxième 17 cavité(s) sont soit vides, soit remplies avec un autre matériau déformable élastiquement différent de celui formant le bloc de matériau 2. Cet autre matériau peut être plus souple que celui du bloc de matériau 2, ou plus rigide. Par exemple, cet autre matériau est une mousse. Dans des variantes de réalisation, le matériau remplissant la première cavité 16 est différent de celui qui remplit la deuxième cavité 17 : ils présentent des durométries différentes. Ceci rend le dispositif dentaire 1 selon l'invention modulable.

La présence du ou des bossages 14,15,34,35, permet, lors d'une utilisation du dispositif dentaire 1 selon l'invention pour une rééducation d'un patient, à ce que les molaires devant lesquelles s'étend en regard le bossage viennent en premier lieu en contact avec ce bossage, lors d'un mouvement de rapprochement des maxillaires inférieur et supérieur. En réaction, le dispositif dentaire 1 selon l'invention va se déformer élastiquement au niveau dudit bossage et appliquer des efforts de réaction sur les maxillaires. Cela à pour conséquence d'obliger une modification du comportement des muscles maxillaires situés du coté du bossage considéré. Ainsi, le dispositif dentaire 1 selon l'invention oblige le patient à une mastication consciente qui le conduit à effectuer un travail actif en autostimulation. Le dispositif dentaire 1 selon l'invention permet une sollicitation optimale des dents, favorise un travail musculaire équilibré des muscles de la mâchoire et procure une détente et un bon fonctionnement des articulations temporo-mandibulaires. D'autre la configuration des première 30 et deuxième 30 gouttières permet au dispositif dentaire 1 selon l'invention de fonctionner comme un guide dentaire. Le dispositif dentaire 1 selon l'invention permet à un patient de réaliser des exercices de mastication, de mouvements latéraux et/ou d'avant/arrière du maxillaire inférieur par rapport au maxillaire supérieur, conduisant à une bonne rééducation de la mâchoire, du fait du caractère dynamique induit du dispositif dentaire 1 selon l'invention.

D'autre part, le dispositif dentaire 1 selon l'invention comporte un orifice antérieur 23 aménagé dans l'épaisseur du fond 31 de la forme en « U » du bloc de matériau 2. L'orifice antérieur 23 est traversant le fond 21. Cet orifice antérieur présente un premier rôle similaire à celui des première 16 et deuxième 17 cavités précédemment décrites : celui de pouvoir adapter la déformabilité du fond 21 lorsque la mâchoire d'un patient est fermée, les incisives des maxillaires supérieur et inférieur étant en appui sur le fond 33,13 respectif des première 30 et deuxième 10 gouttières. De même que pour les cavités 16,17, l'orifice antérieur 23 peut être rempli avec un autre matériau déformable élastiquement différent de celui formant le bloc de matériau 2 ou de ceux remplissant les cavités 16,17. Cet autre matériau peut être plus souple que celui du bloc de matériau 2 ou des cavités 16,17, ou plus rigide. Par exemple, cet autre matériau est une mousse. Ceci rend le dispositif dentaire 1 selon l'invention encore plus modulable et donc adaptable au patient.

L'orifice antérieur 23 présente un deuxième rôle : vide, il peut servir d'orifice de ventilation pour l'utilisateur. Cela permet une utilisation du dispositif dentaire 1 selon l'invention par un patient lors d'une activité sportive par exemple, en permettant une respiration par la bouche.

Dans une variante de réalisation, le dispositif dentaire 1 selon l'invention, comporte un bossage supplémentaire s'étendant en saillie depuis le fond 33,13, de la première 30 et/ou de la deuxième 10 gouttières, au droit de l'orifice antérieur 23. Ce bossage supplémentaire est similaires aux bossages 14,15,34,35 précédemment décrit.

De plus, lorsque le patient mord le dispositif dentaire 1 selon l'invention et que tous les bossages sont totalement écrasés (contact parfait entre le fond des première et deuxième gouttières et les dents des maxillaires supérieur et inférieur), l'épaisseur du matériau du bloc de matériau 2 au niveau du fond 21 de la forme en « U » est plus importante que celle des branches 22 latérales de la forme en « U ». Ceci confère une dimension verticale plus importante à l'avant au dispositif dentaire 1 selon l'invention et permet une meilleure amplitude de l'articulation temporo-mandibulaire et une meilleure efficacité masticatoire.

Dans une variante de réalisation, le dispositif dentaire 1 selon l'invention comporte des bossages additionnels au niveau du fond 21 de la forme en « U » présentant une épaisseur similaire à celle des bossages 14,15,34,35.

Enfin, il est possible d'utiliser le dispositif dentaire 1 selon l'invention de manière passive. Les bossages 14,15,34,35 et le bossage supplémentaire au droit de l'orifice antérieur 23 ont alors une particularité passive d'écarter les maxillaires, de détendre ainsi les articulations temporo-mandibulaires et d'équilibrer les mâchoires (effet ressort).

Le dispositif dentaire 1 selon l'invention peut donc être utilisé au repos pour contrer le bruxisme et la malocclusion dentaire et en dynamique pour rééduquer les muscles et la mâchoire.

Bien entendu, il est possible d'apporter à l'invention de nombreuses modifications sans pour autant sortir du cadre de celle-ci.

## Revendications

1. Dispositif dentaire, destiné à être inséré dans la bouche d'un patient entre des maxillaires supérieur et inférieur, comprenant un bloc de matériau (2) déformable élastiquement, en forme en « U », comportant :
sur une face supérieure, une première gouttière (30) s'étendant le long de la forme en « U », conformée à recevoir en appui les dents du maxillaire supérieur;
sur une face inférieure, une deuxième gouttière (10) s'étendant le long de la forme
en « U », conformée à recevoir en appui les dents du maxillaire inférieur,
l'une parmi les première et deuxième gouttières comportant un premier bossage (34) s'étendant en saillie depuis un fond de ladite gouttière, le bloc de matériau (2) déformable étant en outre en forme de coin s'affinant vers les extrémités des branches de la forme en « U », **caractérisé en ce que** le dispositif comporte une cavité (16) située sous le bossage, entre le bossage et un fond de l'autre parmi les première et deuxième gouttières.

2. Dispositif selon la revendication 1, **caractérisé en ce que** le premier bossage (34) est situé dans l'une des branches de la forme en « U ».

3. Dispositif selon la revendication 2, **caractérisé en ce que** la cavité est remplie avec un deuxième matériau déformable élastiquement différent de celui du bloc déformable élastiquement.

4. Dispositif selon l'une des revendications 1 à 3, **caractérisé en ce que** l'autre parmi les première et deuxième gouttières comporte un deuxième bossage (35) s'étendant en saillie depuis un fond de ladite gouttière, au droit du premier bossage.

5. Dispositif selon l'une des revendications 2 à 4, **caractérisé en ce qu'**un autre bossage est situé dans l'autre des branches de la forme en « U ».

6. Dispositif selon la revendication 5, **caractérisé en ce qu'**une cavité est agencée sous l'autre bossage.

7. Dispositif selon l'une des revendications 1 à 6, **caractérisé en ce qu'**il comporte un orifice antérieur (23) aménagé traversant dans le bloc au niveau d'un fond de la forme en « U » du bloc (2).

8. Dispositif selon la revendication 7, **caractérisé en ce que** l'orifice antérieur (23) est rempli avec un troisième matériau déformable élastiquement différent de celui du bloc déformable élastiquement.

9. Dispositif selon l'une des revendications 1 à 8, **caractérisé en ce que** le bossage s'étend en saillie depuis un fond de ladite gouttière et est situé au niveau d'un fond de la forme en « U ».

10. Dispositif selon l'une des revendications 1 à 9, **caractérisé en ce qu'**il comporte en outre un voile de palais (36) s'étendant depuis un bord de la gouttière supérieure.

11. Dispositif selon l'une des revendications 1 à 10, **caractérisé en ce que** le bloc (2) est réalisé en un élastomère, notamment thermoplastique.

## Patentansprüche

1. Dentalvorrichtung, die dafür vorgesehen ist, in den Mund eines Patienten zwischen Ober- und Unterkiefer eingesetzt zu werden, und aus einem elastisch verformbaren, U-förmigen Materialblock besteht, mit:
an einer Oberseite einer ersten Rinne (30), die sich über die U-Form erstreckt und für eine Stützung durch die Zähne des Oberkiefers ausgelegt ist;
an einer Unterseite einer zweiten Rinne (10), die sich über die U-Form erstreckt und für
eine Stützung durch die Zähne des Unterkiefers ausgelegt ist;
wobei eine dieser beiden Rinnen eine erste Erhebung (34) aufweist, die über die gesamte Länge von einem Grund besagter Rinne aus hervortritt, wobei der Block aus verformbarem Material überdies die Form eines Keils besitzt, der sich zu den Enden der Zweige der U-Form verjüngt und **dadurch gekennzeichnet ist, dass** die Vorrichtung einen Hohlraum (16) aufweist, der unter der Erhebung, zwischen dieser und einem Grund der jeweils anderen Rinne angeordnet ist.

2. Vorrichtung gemäß Patentanspruch 1, die **dadurch gekennzeichnet ist, dass** die erste Erhebung (34) in einem der Zweige der U-Form angeordnet ist.

3. Vorrichtung gemäß Patentanspruch 2, die **dadurch gekennzeichnet ist, dass** der Hohlraum mit einem zweiten elastisch verformbaren Material gefüllt ist, das sich von demjenigen des elastisch verformbaren Blocks unterscheidet.

4. Vorrichtung gemäß einem der Patentansprüche 1 bis 3, die **dadurch gekennzeichnet ist, dass** die jeweils andere Rinne eine zweite Erhebung (35) aufweist, die sich von einem Grund besagter Rinne aus passgenau zur ersten Erhebung erstreckt.

5. Vorrichtung gemäß einem der Patentansprüche 2 bis 4, die **dadurch gekennzeichnet ist, dass** eine weitere Erhebung im anderen der Zweige der U-Form angeordnet ist.

6. Vorrichtung gemäß Patentanspruch 5, die durch gekennzeichnet ist, dass unter der weiteren Erhebung ein Hohlraum angeordnet ist.

7. Vorrichtung gemäß einem der Patentansprüche 1 bis 6, die durch das Vorhandensein einer vorderen Öffnung (23) gekennzeichnet ist, die in Höhe eines Grunds der U-Form des Blocks (2) durch den Block hindurch verläuft.

8. Vorrichtung gemäß Patentanspruch 7, die **dadurch gekennzeichnet ist, dass** die vordere Öffnung (23) mit einem dritten elastisch verformbaren Material gefüllt ist, das sich von demjenigen des elastisch verformbaren Blocks unterscheidet.

9. Vorrichtung gemäß einem der Patentansprüche 1 bis 8, die **dadurch gekennzeichnet ist, dass** die Erhebung sich von einem Grund besagter Rinne aus in Höhe eines Grunds der U-Form erstreckt.

10. Vorrichtung gemäß einem der Patentansprüche 1 bis 9, die **dadurch gekennzeichnet ist, dass** sie überdies ein Gaumensegel (36) aufweist, das sich von einem Rand der oberen Rinne aus erstreckt.

11. Vorrichtung gemäß einem der Patentansprüche 1 bis 10, die **dadurch gekennzeichnet ist, dass** der Block (2) aus einem Elastomer gefertigt ist, das insbesondere thermoplastische Eigenschaften aufweist.

## Claims

1. Dental device designed to be inserted in the mouth of a patient between the maxilla and mandible, comprising a U-shaped elastically deformable block of material (2), comprising:
on an upper face a first trough (30), extending along the U-shaped part, shaped to receive the teeth of the maxilla pressed against it;
on a lower face a second trough (10), extending along the U-shaped part, shaped to receive the teeth of the mandible pressed against it,
one among the first and second troughs comprising a first boss (34) extending jutting out from the bottom of said trough, the block of material (2) also being wedge-shaped narrowing towards the ends of the branches of the U-shape, **characterised by** the fact the device comprises a cavity (16) situated under the boss, between the boss and one bottom of the other of the first and second troughs.

2. Device as per claim 1, **characterised by** the fact the first boss (34) is situated in one of the branches of the U-shape.

3. Device as per claim 2, **characterised by** the fact the cavity is filled with a second elastically deformable material different from that of the elastically deformable block.

4. Device as per one of the claims 1 to 3, **characterised by** the fact that the other of the first and second troughs comprises a second boss (35) extending jutting out from a bottom of said trough, at the level of the first boss.

5. Device as per one of the claims 2 to 4, **characterised by** the fact another boss is situated in the other branch of the U-shape.

6. Device as per claim 5, **characterised by** the fact that a cavity is made under the other boss.

7. Device as per one of the claims 1 to 6, **characterised by** the fact that it comprises an anterior orifice (23) made passing through the block at the level of a bottom of the U-shape of block (2).

8. Device as per claim 7, **characterised by** the fact that the anterior orifice (23) is filled with a third elastically deformable material different from that of the elastically deformable block.

9. Device as per one of the claims 1 to 8, **characterised by** the fact that the boss extends jutting out from the bottom of said trough and is situated at the level of a bottom of the U-shape.

10. Device as per one of the claims 1 to 9, **characterised by** the fact it also comprises a palate (36) extending from one edge of the upper trough.

11. Device as per one of the claims 1 to 10, **characterised by** the fact that the block (2) is made of an elastomer, notably thermoplastic.
